(19)

Europäisches
Patentamt
European
Patent Office
Office européen
des brevets

(11) **EP 2 082 688 A1**

(12) **EUROPEAN PATENT APPLICATION**

(43) Date of publication:
**29.07.2009 Bulletin 2009/31**

(51) Int Cl.:
***A61B 8/00*** *(2006.01)*     ***G06T 7/20*** *(2006.01)*
***G01S 7/52*** *(2006.01)*

(21) Application number: **08169745.0**

(22) Date of filing: **24.11.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **23.01.2008 US 11178**

(71) Applicant: **Siemens Medical Solutions USA, Inc.
Malvern, PA 19355 (US)**

(72) Inventors:
• **Guracar, Ismayil M**
 **Redwood City, CA 94062 (US)**
• **Houle, Helen**
 **Sunnyvale, CA 94087 (US)**
• **Lee, Chi-Yin**
 **Bellevue, WA 98007 (US)**

(74) Representative: **Hazzard, Alan David
Siemens AG
Postfach 22 16 34
80506 Munich (DE)**

(54) **Synchronized combining for contrast agent enhanced medical diagnostic ultrasound imaging**

(57) Contrast agent enhanced medical diagnostic imaging is provided. Frames of data from common phase periods are grouped (32). Motion correction (34) is performed within each common phase group. An image representing contrast agents is formed (36) from a combination of the frames within each common phase, motion corrected group.

Figure 1

**Description**

BACKGROUND

The present embodiments relate to contrast agent enhanced medical diagnostic ultrasound imaging. In particular, contrast agent image information is combined over time.

Contrast echocardiography is a widely used technique in imaging the heart. One of the primary applications is left ventricular opacification (LVO) in which the contrast microbubbles opacify the left ventricle and thus provide better visualization of the endocardial border. An evolving application of contrast echo is myocardial contrast echocardiography (MCE) in which the contrast microbubbles provide traces for the microcirculation and for myocardial perfusion. Imaging blood perfusion in organs or tissue may be useful. In some applications, frames of data acquired over time are integrated. The resulting image may provide useful information for diagnosis, such as showing smaller vessels or perfusion channels.

Some example combinations are maximum intensity holding/processing (MIP), minimum intensity holding, and the construction of a time intensity curve (TIC). Maximum intensity processing combines the high luminance contrast portion over time. TIC charts intensity (e.g., contrast agent frame intensity) for a pixel or region of interest as a function of time. The chart shows the in-flow, out-flow, or both of contrast agents over the time associated with the component frames of data. However, due to operator motion or internal motion, the combination of information from different frames may result in blurred images or inaccurate information. Motion correction between frames of a sequence may not fully correct for blurring since the imaged tissue may move relative to other tissue.

BRIEF SUMMARY

By way of introduction, the preferred embodiments described below include methods, systems, computer readable media, and instructions for contrast agent enhanced medical diagnostic imaging. Frames of data from common phase periods are grouped. Motion correction is performed within each common phase group. An image representing contrast agents is formed from a combination of the frames within each common phase, motion corrected group.

In a first aspect, a method is provided for contrast agent enhanced medical diagnostic ultrasound imaging. A sequence of ultrasound frames of data is generated. The frames of data represent, at least in part, information from contrast agents. The sequence is over a plurality of heart cycles. A first subset of the sequence is selected as ultrasound frames of data corresponding to a substantially same phase of the heart cycle. The ultrasound frames of data of the first subset corresponding to the substantially same phase of the heart cycle are corrected for motion. An image is formed as a function of the motion corrected ultrasound frames of data of the first subset. The image represents, at least in part, the information from contrast agents at the phase of the heart cycle.

In a second aspect, a computer readable storage medium has stored therein data representing instructions executable by a programmed processor for contrast agent enhanced medical diagnostic ultrasound imaging. The storage medium includes instructions for obtaining frames of contrast agent data and frames of B-mode data, the frames of contrast agent and B-mode data corresponding to a first phase of a cardiac cycle over a plurality of cardiac cycles, correcting for motion between the frames of contrast agent data as a function of the frames of B-mode data, then combining the contrast agent data corresponding to the first phase, and generating an image as a function of the combined contrast agent data.

In a third aspect, a system provides enhanced medical diagnostic ultrasound imaging of contrast agents. An ECG input is operable to receive cardiac cycle information. A processor is operable to spatially align frames of data output from a contrast agent detector as a function of frames of data output from a B-mode detector. The spatial alignment is for frames of data output from the contrast agent detector with cardiac cycle information indicating a substantially same time within a cardiac cycle. The processor is operable to combine the spatially aligned frames of data output from the contrast agent detector and having the cardiac cycle information indicating the substantially same time within the cardiac cycle.

The present invention is defined by the following claims, and nothing in this section should be taken as a limitation on those claims. Further aspects and advantages of the invention are discussed below in conjunction with the preferred embodiments.

BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.

Figure 1 is a block diagram of one embodiment of an ultrasound imaging system for contrast agent enhanced imaging;

Figure 2 is a flow chart diagram of a method for contrast agent enhanced diagnostic medical ultrasound imaging according to one embodiment;

Figure 3 is a graphical representation of selecting frames of data corresponding to a same phase of a heart cycle from a sequence of frames; and

Figure 4 is a graphical representation of motion correction across frames of data with common heart cycle phase.

DETAILED DESCRIPTION OF THE DRAWINGS AND PRESENTLY

PREFERRED EMBODIMENTS

A processor extracts frames of contrast agent data at the same cardiac phase across different cardiac cycles. The frames of contrast agent data at a given cardiac phase are aligned. The contrast agent data may be aligned using B-mode data. The aligned contrast agent frames at a same cardiac phase over different heart cycles are combined. Moving average, MIP, or parametric calculation, such as time to peak, is applied to the motion corrected contrast agent frames.

Left Ventricle Opacification (LVO) and Myocardial Contrast Echocardiography (MCE) are two primary applications for Contrast Echo. These applications may be improved by motion corrected imaging from frames with a common phase of the heart cycle. Speckle noise may be reduced, and signal-to-noise ratio may increase. Contrast resolution and visibility of micro-vessels may be increased.

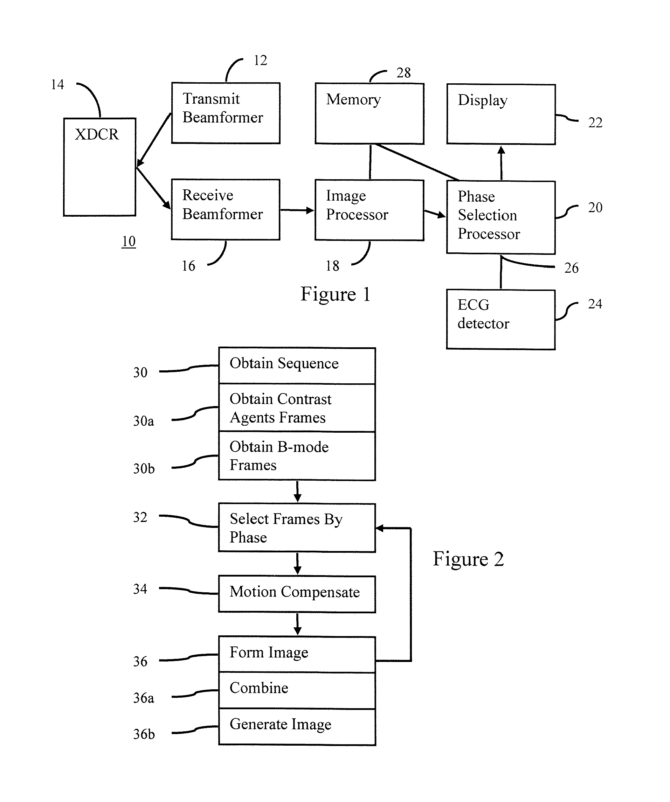

Figure 1 shows a system 10 for enhanced contrast agent medical diagnostic ultrasound imaging. The system 10 includes a transmit beamformer 12, a transducer 14, a receive beamformer 16, an image processor 18, a selection processor 20, a display 22, an ECG device 24, an ECG input 26, and a memory 28. Additional, different, or fewer components may be provided. For example, a separate memory is provided for buffering or storing frames of data. As another example, the selection processor 20 is combined with or part of the image processor 18. In another example, the ECG device 24 is not provided and the ECG input 26 is an output from the image processor 18 or other processor.

The system 10 is a medical diagnostic ultrasound imaging system in one embodiment, but other imaging systems of the same (ultrasound) or different modality may be used. The system 10 provides real-time or offline operation. In other embodiments, part or all of the system 10 is implemented in a computer or workstation. For example, previously acquired frames of data are processed without the beamformers 12, 16 or transducer 14. Offline software on the computer or workstation implements the common phase motion correction for contrast agent imaging.

The ECG detector 24 includes a processor and one or more electrode leads. The heart cycle of a patient is detected. The heart cycle, trigger events, or other characteristics of the heart cycle are output to the ECG input 26. Received cardiac cycle information is used to identify frames of data associated with specific times within the heart cycle. In other embodiments, the ECG input 26 is provided as part of the processor 18 or 20 by analyzing ultrasound data.

The transmit beamformer 12 is an ultrasound transmitter, memory, pulser, analog circuit, digital circuit, or combinations thereof. The transmit beamformer 12 is operable to generate waveforms for a plurality of channels with different or relative amplitudes, delays, and/or phasing. Upon transmission of acoustic waves from the transducer 14 in response to the generated waves, one or more beams are formed. The transmit beamformer 12 may cause the beam to have a particular phase and/or amplitude. For example, the transmit beamformer 12 transmits a sequence of pulses associated with a given scan line or to adjacent scan lines. The pulses correspond to beams with different amplitudes and/or relative phases. In alternative embodiments, a single beam is used for any given scan line and/or beams with a same amplitude and/or relative phases are used.

The transducer 14 is a 1-, 1.25-, 1.5-, 1.75- or 2-dimensional array of piezoelectric or capacitive membrane elements. The transducer 14 includes a plurality of elements for transducing between acoustic and electrical energies. The elements connect with channels of the transmit and receive beamformers 12, 16.

The receive beamformer 16 includes a plurality of channels with amplifiers, delays, and/or phase rotators, and one or more summers. Each channel connects with one or more transducer elements. The receive beamformer 16 applies relative delays, phases, and/or apodization to form one or more receive beams in response to each transmission. In alternative embodiments, the receive beamformer 16 is a processor for generating samples using Fourier or other transforms.

The receive beamformer 16 may include a filter, such as a filter for isolating information at a second harmonic or other frequency band relative to the transmit frequency band. Such information may more likely include desired tissue, contrast agent, and/or flow information. In another embodiment, the receive beamformer 16 includes a memory or buffer and a filter or adder. Two or more receive beams are combined to isolate information at a desired frequency band, such as a second harmonic, cubic fundamental or other band.

Any desired sequence of transmit and receive operation may be used to obtain ultrasound information. For example, B-mode data may be obtained by scanning a region once. The B-mode data may be used for tissue imaging. Correlation or motion tracking may be used to derive fluid information from B-mode data. B-mode operation may provide contrast agent information. Doppler information may be obtained by transmitting sequences of beams along each scan line. A corner turning memory may be used to isolate tissue, contrast agents, and/or flow information from Doppler signals. Other now known or later developed modes may be used.

In one embodiment, the mode is a contrast agent imaging mode. Contrast agents may be imaged with typical B-mode or Doppler techniques. Isolating information at the second, even, odd, sub, or other harmonics may more likely identify information from contrast agents. For example, a two pulse technique is used. The pulses have a same amplitude, but different phase. By summing the response, information associated with even harmonics is identified. Filtering may alternatively be used. Alternatively or additionally, relative phasing is provided in the receive processing.

In one embodiment, the transmit sequence is controlled to generate echo signals responsive to the cubic fundamental. The beamformer 12 is operable to transmit a plurality of pulses having at least two different amplitude levels and at least two of the plurality of pulses having opposite or different phases. Transmitter power can be varied in any suitable manner, as for example by adjusting the voltage applied to individual transducer elements, or by adjusting the number of transducer elements (or transmit aperture) used to form a particular pulse.

For obtaining ultrasound data at the cubic fundamental, the receive beamformer 16 includes line memories and a summer or a filter to combine signals responsive to the transmissions. The line memories or buffers can be formed as physically separate memories, or alternately they can be formed as selected locations in a common physical device. The beamformed signals are stored in the line memories or buffers and then weighted and summed in a weighted summer. Weighting values for both amplitude and phase are used in the weighted summer. The memories and the summer can be implemented using analog or digital techniques. The weighted summer forms a composite output signal by weighting the separate beamformed receive signals. The composite output signal for a given spatial location is a sample associated with the cubic fundamental response.

Obtaining cubic fundamental information is disclosed in U.S. Patent No. 6,494,841, the disclosure of which is incorporated herein by reference. Any of the transmit sequences and receive combinations disclosed therein may be used for obtaining cubic fundamental information. Other transmit sequences and receive combinations for obtaining cubic fundamental information may be used, such as disclosed in U.S. Patent Nos. 6,602,195, 6,632,177, 6,638,228 and 6,682,482, the disclosures of which are incorporated herein by reference. In general, a sequence of pulses with different amplitudes and phases are transmitted. Using amplitude change or different amplitudes without different phases may also be used to obtain cubic fundamental information. By combining received signals responsive to the sequence, a sample including cubic fundamental information is obtained. The cubic fundamental information is highly specific to ultrasound contrast agents since contrast agents produce cubic response and the transducer and tissue produce very little cubic response. The information provides tissue clutter rejection, allowing for imaging more specific to contrast agents. For example, small vessels within tissue may be more easily imaged or identified using cubic fundamental information.

The image processor 18 is a B-mode detector, Doppler detector, pulsed wave Doppler detector, contrast agent detector, correlation processor, Fourier transform processor, application specific integrated circuit, general processor, control processor, field programmable gate array, digital signal processor, analog circuit, digital circuit, combinations thereof, or other now known or later developed device for detecting information for display from beamformed ultrasound samples.

In one embodiment of a contrast agent detector, the image processor 18 implements a fast Fourier transform from a plurality of samples representing a same region or gate location. Each of the samples is responsive to cubic fundamental so that a pulsed wave Doppler display may be generated from cubic fundamental information. Any of the contrast agent detectors in the patents reference above may be used. Other components may be used for a contrast agent detector. For example, B-mode detection is provided. As another example, a filter combines information from different transmissions to enhance or better isolate the response from contrast agents (e.g., second harmonic or cubic fundamental). The filter obtains information primarily at a cubic fundamental or other frequency band of the transmitted ultrasound signals. Any detection of the signals is then performed.

The image processor 18 also includes a B-mode detector in a parallel track. The B-mode detector operates on the same or different beamformed samples to detect tissue, contrast agent, or tissue and contrast agent response. For example, one receive beam for each spatial location from the sequence of receive beams used for cubic fundamental isolation is applied to the B-mode detector for imaging primarily tissue information.

The image processor 18 outputs frames of ultrasound data. The frames of data are formatted in an acquisition format (e.g., polar coordinate), a display format (e.g., scan converted into a Cartesian coordinate format or an image), or other format. Each frame of data represents a one, two, or three-dimensional scanned region, such as substantially the entire region to be imaged (substantially accounting for patient or transducer motion). The frames of data include a single or multiple types of data. For example, one frame of data includes just contrast agent information. As another example, one frame of data includes contrast agent information for some spatial locations and another type of information (e.g., B-mode or Doppler) for other spatial locations. Different types of data may be provided in the same frame for a same spatial location. In another example, the different types of data are provided in different frames of data.

In an alternative embodiment, the image processor 18 loads data from a network or memory. For example, to acquire or obtain ultrasound data, DICOM or other images are loaded. Each image is a frame of data. One frame may include different types of data, one overlaid on another. Alternatively, each frame includes only one type of data with different frames for different data types. In another embodiment, each frame is subdivided so that one portion includes one type of data and another portion includes another type of data.

The selection processor 20 is an application specific integrated circuit, correlation processor, Fourier transform processor, general processor, control processor, field programmable gate array, digital signal processor, analog circuit, digital circuit, combinations thereof, or other now known or later developed device for determining similarity and/or displacement between frames of data.

The selection processor 20 receives the frames of data to determine which frames should be included in MIP, TIC, or other images generated from combinations of information from frames of data. The selection processor 20 selects as a function of the phase associated with each frame of data. As the frames of data are acquired, a phase relative to the heart cycle is determined for the frame of data. Frames of data output from the contrast agent detector with cardiac cycle information indicating a substantially same phase within a cardiac cycle are selected for each group. For example, a plurality of groups of frames is created where each group is for a different phase.

The selection processor 20 spatially aligns frames of data output from the contrast agent detector. The frames of data of each group or for each phase are aligned with each other. The frames of data may be spatially aligned based on external sensors, such as transducer position sensors. The frames of data may be spatially aligned based on the contrast agent data. In one embodiment, the frames of contrast agent data are spatially aligned as a function of frames of data output from the B-mode or other non-contrast agent detector. The B-mode frames are acquired at the same or similar times as the contrast agent frames, such as in a line or frame interleave. Aligning the B-mode frames indicates alignment of the contrast agent frames acquired at the same or similar times.

The selection processor 20 may also include a persistence filter, other filter, summer, alpha blending buffer, other buffer, memory, processor, adder, or other device for generating an image from information of different frames of data. The selection processor 20 combines the spatially aligned frames of data output from the contrast agent detector. Cardiac cycle information indicates that the frames to be combined have a substantially same time within the cardiac cycle. For example, the selection processor 20 compares data for a particular spatial location from one frame to another frame or an ongoing combination frame.

Based on the comparison (e.g., highest value, contribution to mean value, or lowest value), one of the values is selected or the ongoing combination frame is updated to include the desired value. As another example, the selection processor 20 determines an average, total, or other value representing a location or region as a function of time. Combinations of imaging types may be used.

The display 20 is a CRT, monitor, LCD, flat panel, projector or other display device. The display 20 receives display values for displaying an image. The display values are formatted as a one-dimensional image, two-dimensional image, or three-dimensional representation. In one embodiment, the display values are for an image generated as a function of frames of data acquired at different times, such as a TIC or MIP image. An image of the combined frames of data output from the contrast agent detector is generated. As additional frames of data are acquired and selected, the image may be updated. Other images, such as images from single or component frames of data, may also be displayed.

The image processor 18 and/or selection processor 20 operate pursuant to instructions. The memory 28 is a computer readable memory. A computer readable storage medium stores data representing instructions executable by one or both of these programmed processors for contrast agent enhanced medical diagnostic ultrasound imaging. The instructions for implementing the processes, methods and/or techniques discussed herein are provided on computer-readable storage media or memories, such as a cache, buffer, RAM, removable media, hard drive or other computer readable storage media. Computer readable storage media include various types of volatile and nonvolatile storage media.

The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination. Likewise, processing strategies may include multiprocessing, multitasking, parallel processing and the like. In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU or system.

Figure 2 shows a method for contrast agent enhanced medical diagnostic ultrasound imaging. The method is implemented by the system 10 of Figure 1 or a different system. The method is performed in the order shown or a different order. Additional, different, or fewer acts may be provided, such as not providing act 30b and/or act 36.

In act 30, a sequence of ultrasound frames of data is obtained. The sequence is generated by acquiring frames of data with ultrasound or by acquiring previously generated frames of data (e.g., DICOM images). The frames of data are acquired in real time with live scanning or are from stored clips. The sequence may be substantially continuous or periodic (e.g., acquired once or more every heart cycle).

The sequence includes frames of data representing a scanned region at different times. Each frame of data represents a same or overlapping region. Some frames may represent different regions, such as due to out-of-plane motion of the transducer relative to the patient.

The region includes contrast agents or an area likely to include contrast agents after insertion of the agents. The contrast agents respond to ultrasound energies. Frames of contrast agent data are obtained in act 30a. Some or all of the frames of data include information from contrast agents. The information may also include response from tissue or fluids. In one embodiment, the information is obtained at a cubic fundamental of ultrasound signals. For example, ultrasound signals are transmitted in a plurality of pulses having at least two different amplitude levels and phases. To avoid or minimize destruction of the contrast agents, low amplitude transmissions (e.g., MI less than 0.7) are used. Signals responsive to the transmissions are combined, such as by summing or weighted summing. The combination provides data primarily responsive to contrast agents. Data is acquired at each spatial location of a region of interest in each frame of data.

Only one type of data is represented in the frames of data, such as data representing just contrast agents or responses from contrast agent and tissue. Alternatively, the frames of data represent different types of data, such as in a same frame or in different sets of frames. For example, frames of contrast agent data and separate frames of B-mode data are obtained. Frames of B-mode or tissue information are obtained in act 30b. The B-mode information is generated separately from the contrast agent information. Alternatively, echo signals responsive to one of the pulses (e.g., the full or highest amplitude pulse) used for contrast agent information are used for B-mode detection. The B-mode or tissue information may include other information. For example, pulse sequences and/or filtering provide for tissue information from ultrasound signals at a fundamental, second harmonic, or both.

The frames of contrast agent and B-mode data correspond to phases of a cardiac cycle. Each frame is acquired generally at a give time. The acquired frames are tagged with a time or phase, or other indication of the timing of acquisition relative to the cardiac cycle is recorded. Different frames may be associated with different portions of the cycle. The frames may be acquired using triggering based on the cardiac cycle so that the frames are acquired at desired phases. In cardiac echo imaging, the ECG or R-wave signals are generally available. The ultrasound acquisition may be synchronized with the R-wave using triggered sequence. Alternatively, the frames are acquired without triggering.

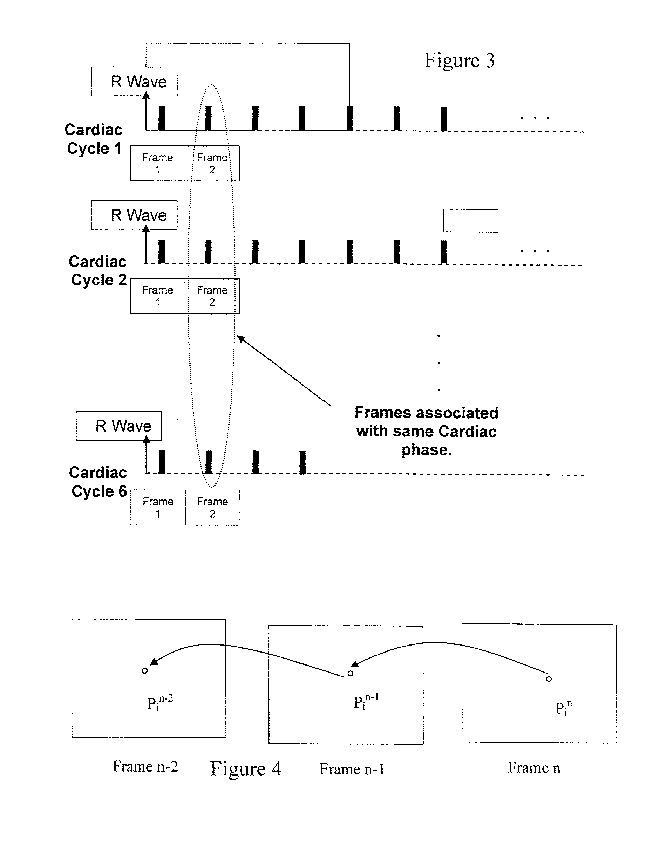

The sequence extends over a plurality of cardiac cycles. Different frames may be acquired during different cycles, but associated with a same phase. Figure 3 shows a plurality of frames (short black bars) acquired during each of six cardiac cycles. Each cycle begins at the R-wave, but may begin at other times. A frame of data is obtained for each of a plurality of different phases during each cycle. In alternative embodiments, the timing does not align exactly. One or more cycles may not have frames of data associated with a given phase. The sequence of ultrasound frames of data represents, at least in part, information from contrast agents.

In act 32, frames for one or more subsets of the sequence are selected. Each subset is populated with frames of data corresponding to a substantially same phase of the heart cycle. Substantially accounts for variation in the heart cycle and/or frames closer to the particular phase than to an adjacent phase of another group. In either a triggered or non-triggered sequence, frames associated with similar cardiac phase over various heart cycles are grouped using frame time stamp and/or the R-wave time stamp. In the example of Figure 3, a dashed oval designates Frame 2 from each cycle in one group or subset of the six-cycle sequence. Other or different subsets may be formed, such as a group for the R-wave or a diastolic period.

Frames within a given subset may be discarded or not used based on other characteristics. For example, the frames of data associated with less inter frame motion are selected, and frames of data associated with more inter frame motion are not selected. The frames of data with undesired motion are discarded. Any desired threshold may be used. Other criteria may be used.

In act 34, frames within each subset are spatially aligned to compensate for motion. The motion correction occurs across the frames associated with the substantially same phase of the heart cycle. For a given phase, the frames are aligned. The frames of each of the different subsets are aligned with other frames within the corresponding subset.

Motion compensation of act 34 may be applied to the frames of data to correct for in-plane motion between frames. In-plane motion may be due to transducer movement, patient movement, and/or movement of the tissue within the region of interest. To compensate for motion, a relative translation and/or rotation along one or more dimensions is determined. Data from one frame is correlated with different regions in the other frame of data to identify a best or sufficient match. Correlation, cross-correlation, minimum sum of absolute differences, and/or another measure of similarity may be used. Global or local motion may be corrected. For example, the motion between the frames for a plurality of different regions is determined. A global motion may be determined from the plurality of motion corrections or the motion correction may be applied separately for each region, warping the frame. Rigid or non-rigid motion models may be provided, such as warping in additional to translating and rotating in a non-rigid motion model. The entire frame of data or a window of data may be used for determining the best match and corresponding motion.

For each new frame of data, the previous or temporally adjacent, selected frame of data is used as the reference frame. Alternatively, the same reference frame is used for comparison to each subsequent, even temporally spaced, frames of data.

The displacement of the data between frames is then used to align the spatial locations between frames. The motion correction may remove or lessen motion associated with transducer movement, patient movement, or organ movement. Alternatively, no motion correction between frames is used.

Motion sensors may be used to determine motion compensation. For ultrasound data based correction, the frames of ultrasound data may be used. In one embodiment, frames of B-mode data are used to determine the alignment of the frames of contrast agent data. The correction is performed as a function of the B-mode data. Given the frames associated with a same phase, the corresponding frames of B-mode or tissue response data are aligned by estimating the motion parameters between them using a rigid motion or non-rigid motion model. Figure 4 shows a point $P_i^n$ in the $n^{th}$ frame, the corresponding position of this point is recovered as $P_i^{n-1}$ in the $(n-1)^{th}$ frame, ..., and $P_i^1$ in the 1st frame, respectively through frame motion estimation. The similarity between B-mode or tissue frames indicates the alignment of the B-mode or tissue frames. Where the aligned frames are acquired in the same cycles and phase as the contrast agent frames, the alignment of the B-mode or tissue frames also indicates a spatial alignment of the contrast agent frames. By motion correcting using B-mode, errors in alignment due to contrast agent motion may be avoided. Since contrast agents may be moving, motion correction based on contrast agent response may be inaccurate.

In act 36, an image is formed. The image is formed as a function of the motion corrected ultrasound frames of data of a subset or common phase. The image represents, at least in part, the information from contrast agents at the phase of the heart cycle. Blurring may be limited or avoided by motion correction. The image may be formed once or updated as more frames associated with the same phase are acquired. Images for other subsets or phases may be generated. Other images, such as B-mode images, may be generated. The images are grayscale, color, or combinations thereof.

In act 36a, information from the selected subset of frames and not from unselected ones of the ultrasound frames of data is combined. A new frame of data or image is generated as a function of data from the selected frames. The selected frames of ultrasound data are integrated as a function of time. Integration includes mathematical integration or forming an image from a plurality of sources. By combining information from contrast agents, such as information primarily at a cubic fundamental of ultrasound signals, the perfusion of contrast agents and/or small vasculature may be viewed more easily.

For each spatial location of a region of interest or all the spatial locations represented by the frames of the subset, the data is compared or used to determine a value. For each pixel of the image, a value is selected as a function of data from each of the remaining (selected) frames of data with common phase. The combination is for any now known or later developed inter-frame processing, such as maximum intensity holding, minimum intensity holding, mean determination, or constructing one or more time intensity curves.

For example, the mean, median or other statistical value of data is determined from the frames for each spatial location as a function of time. In one embodiment, the contrast agent data is combined by averaging the frames of contrast agent data corresponding to the first phase. The ultrasound frames of data of a selected subset are averaged, such as with a weighted moving average. Consider $I(P_i^n)$ to be the intensity of point $P_i^n$ in the $n^{th}$ frame. The weighted moving average of length k (k points weighted moving average) for point $P_i^n$ is computed as follows:

$$MovingAverage(I(P_i^n)) = \frac{(w_n I(P_i^n) + w_{n-1} I(P_i^{n-1}) + \ldots\ldots + w_{n-k+1} I(P_i^{n-k+1}))}{(w_n + w_{n-1} + \ldots + w_{n-k+1})}$$

where $w_n$, $w_{n-1}$, ..., $w_{n-k+1}$ are weighting coefficients. The moving window defines a number of the most recently acquired frames of the subset to include in the average, such as the most recent three frames of data. For example, a three frame moving average is provided for a subset of frames corresponding to the end diastolic portion of the heart cycle. Infinite or finite impulse response averaging may be used. In an alternative embodiment, a weighted average of all of the frames of the subset of common phase is provided.

Temporal averaging can provide noise reduction for the image sequence in general. In contrast enhanced ultrasound image sequence, the speckle pattern at a given location may change due to the motion of the contrast agents. As a result, temporal averaging may reduce the speckle noise in this respect.

As another example combination, the maximum, minimum, or other data in relation to data of the selected frames is selected based on comparison. The frames of the selected subset are combined into a persisted frame or single frame. For example, a maximum intensity projection frame of data is formed from the frames of contrast agent data. A maximum of the information from contrast agents is selected for each spatial location of the image or a region of interest. Consider $I(P_i^n)$ be the intensity of Point $P_i^n$ in the $n^{th}$ frame. The maximum intensity projection (along the time axis) of point i is computed as follows:

$$MIP(I(P_i^n)) = \max(I(P_i^n), I(P_i^{n-1}), I(P_i^{n-2}), ...., I(P_i^1))$$

The maximum intensity projection is performed over the subset or a moving window of motion corrected frames representing a same or similar phase of the heart cycle.

In myocardial contrast echocardiography, contrast agents show the time trace of the myocardial perfusion. Since the contrast agents are moving over time, it is desirable to integrate all these time variant information together for better depiction of the myocardial perfusion.

In another example of combination, a curve representing intensity or other contrast agent response as a function of time is determined from the frames. The curve is for a region or for a spatial location. Since the frames are associated with different times, the curve is of intensity as a function of time.

In act 36b, the image is generated as a function of the combination. The frame of data formed by combining data from other frames is scan converted, color coded, mapped, and/or converted into a display format. For example, the contrast agent data is color mapped. The combination may be combined with other ultrasound information, such as being overlaid on a tissue image. Red, blue, green (RGB) or other display values are formed from the contrast agent data. The resulting image represents contrast agents at a heart cycle phase after spatial alignment.

As represented by the feedback from act 36 to act 32, the method may be repeated. In the embodiment shown, the selecting, motion compensation, combining, and image generation are repeated. The obtaining may or may not be repeated. For example, the frames of data represented in Figure 3 are acquired in act 30. Rather than repeat act 30, the selection act 32 is repeated for different phases. After correction for motion in act 34, images are formed for each of the phases or subsets corresponding to different phases. As another example, an image associated with a given phase is updated as more frames of data are acquired.

In one example, B-mode imaging is performed with left ventricular opacification. The Sequoia c512 Ultrasound machine and the 4V1c transducer from Siemens Medical Solutions, USA image with a Contrast Pulse Sequencing technique (CPS - cubic fundamental). Ten beat, Apical 4 chamber cine clips are acquired. These DICOM movies are processed offline with software. The software temporally synchronizes the beats and performs a weighted moving average of three beats over the ten beat sequences. Maximum intensity projection is also applied to the acquired movies. Good left ventricular endocardial blood interface may be visualized. Improved signal to noise ratio improvement as well as contrast resolution enhancements may be provided.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the spirit and scope of this invention.

**Claims**

1. A method for contrast agent enhanced medical diagnostic ultrasound imaging, the method comprising:

generating (30) a sequence of ultrasound frames of data representing, at least in part, information from contrast agents, the sequence being over a plurality of heart cycles;
selecting (32) a first subset of the sequence, the first subset being ultrasound frames of data corresponding to a substantially same phase of the heart cycle;
correcting (34) for motion between the ultrasound frames of data of the first subset corresponding to the substantially same phase of the heart cycle; and
forming (36) an image as a function of the motion corrected ultrasound frames of data of the first subset, the image representing, at least in part, the information from contrast agents at the phase of the heart cycle.

2. The method of Claim 1 wherein generating (30) comprises obtaining (30a) the data as information at a cubic fundamental of ultrasound signals.

3. The method of Claim 2 wherein obtaining (30a) comprises transmitting the ultrasound signals in a plurality of pulses having at least two different amplitude levels and phases, and combining (36a) signals responsive to the transmitting.

**4.** The method of Claim 3 further comprising:

generating (30b) B-mode information as a function of echo signals responsive to one of the plurality of pulses;

wherein correcting (34) comprises correcting (34) as a function of similarity between the B-mode information corresponding to the ultrasound frames of data.

**5.** The method of Claim 1 wherein correcting (34) comprises correcting (34) as a function of the B-mode data, and forming (36) comprises combining (36a) the information from contrast agents.

**6.** The method of Claim 1 wherein correcting (34) comprises:

determining a motion displacement between the ultrasound frames of data of the first subset; and
spatially aligning the ultrasound frames of data of the first subset as a function of the motion displacement.

**7.** The method of Claim 1 wherein forming (36) comprises averaging the ultrasound frames of data of the first subset.

**8.** The method of Claim 1 wherein forming (36) comprises selecting, from the ultrasound frames of data of the first subset, a maximum of the information from contrast agents for each spatial location of the image.

**9.** The method of Claim 1 further comprising:

repeating the selecting (32), correcting (34), and forming (36) for a different phase of the heart cycle with a second subset of the sequence, the second subset being ultrasound frames of data corresponding to the different phase of the heart cycle.

**10.** In a computer readable storage medium (28) having stored therein data representing instructions executable by a programmed processor for contrast agent enhanced medical diagnostic ultrasound imaging, the storage medium (28) comprising instructions for:

obtaining (30) frames of contrast agent data and frames of B-mode data, the frames of contrast agent and B-mode data corresponding to a first phase of a cardiac cycle over a plurality of cardiac cycles;
correcting (34) for motion between the frames of contrast agent data as a function of the frames of B-mode data; then
combining (36a) the contrast agent data corresponding to the first phase; and
generating (30) an image as a function of the combined contrast agent data.

**11.** The computer readable storage medium (28) of Claim 10 wherein obtaining (30) frames of contrast agent data comprises obtaining information primarily at a cubic fundamental of ultrasound signals, and wherein obtaining frames of B-mode data comprises obtaining information from the ultrasound signals at a fundamental, second harmonic, or both.

**12.** The computer readable storage medium (28) of Claim 11 wherein obtaining the information primarily at the cubic fundamental comprises transmitting the ultrasound signals in a plurality of pulses having at least two different amplitude levels and phases, and combining (36a) signals responsive to the transmitting, and wherein obtaining the frames of B-mode data comprises generating B-mode information as a function of echo signals responsive to one of the plurality of pulses.

**13.** The computer readable storage medium (28) of Claim 10 wherein correcting (34) comprises:

determining a motion displacement between the frames of B-mode data; and
spatially aligning the frames of contrast agent data as a function of the motion displacement.

**14.** The computer readable storage medium (28) of Claim 10 wherein combining (36a) comprises averaging the frames of contrast agent data corresponding to the first phase.

**15.** The computer readable storage medium (28) of Claim 10 wherein combining (36a) comprises forming (36b) a maximum intensity projection frame of data from the frames of contrast agent data.

**16.** The computer readable storage medium (28) of Claim 10 further comprising:

repeating the obtaining (30), correcting (34), combining (36a), and generating (30) for a second phase of the cardiac cycle different than the first phase.

**17.** A system for enhanced medical diagnostic ultrasound imaging of contrast agents, the system comprising:

an ECG input (26) operable to receive cardiac cycle information;
a contrast agent detector (18);
a B-mode detector (18); and
a processor (20) operable to spatially align frames of data output from the contrast agent detector (18) as a function of frames of data output from the B-mode detector (18), the spatial alignment being for frames of data output from the contrast agent detector with cardiac cycle information indicating a substantially same time within a cardiac cycle, and the processor (20) operable to combine the spatially aligned frames of data output from the contrast agent detector (18) and having the cardiac cycle information indicating the substantially same time within the cardiac cycle.

**18.** The system of Claim 17 wherein the contrast agent detector (18) comprises a filter operable to obtain information primarily at a cubic fundamental of transmitted ultrasound signals, and wherein the B-mode detector (18) is operable to detect tissue information in response to a subset of the transmitted ultrasound signals.

**19.** The system of Claim 17 further comprising an ECG detector (24) connected with the ECG input (26), and a display operable to generate an image of the combined frames of data output from the contrast agent detector.

**20.** The system of Claim 17 wherein the processor (20) is operable to combine the spatially aligned frames of data output from the contrast agent detector as a maximum intensity projection, an average, or combinations thereof.

## Figure 1

XDCR 14

10

Transmit Beamformer 12

Receive Beamformer 16

Memory 28

Image Processor 18

Phase Selection Processor 20

Display 22

26

ECG detector 24

Figure 1

## Figure 2

30 — Obtain Sequence

30a — Obtain Contrast Agents Frames

30b — Obtain B-mode Frames

32 — Select Frames By Phase

34 — Motion Compensate

36 — Form Image

36a — Combine

36b — Generate Image

Figure 2

Figure 3

Frames associated with same Cardiac phase.

Frame n-2     Figure 4     Frame n-1          Frame n

Europäisches
Patentamt

European
Patent Office

Office européen
des brevets

**PARTIAL EUROPEAN SEARCH REPORT**     Application Number

which under Rule 63 of the European Patent Convention EP 08 16 9745
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 6 659 953 B1 (SUMANAWEERA THILAKA S [US] ET AL) 9 December 2003 (2003-12-09) * column 2, line 30 - column 9, line 56 * * figures 1,2 * ----- | 10-20 | INV. A61B8/00 G06T7/20 G01S7/52 |
| Y | US 2005/033179 A1 (GARDNER EDWARD A [US] ET AL) 10 February 2005 (2005-02-10) * abstract * * paragraph [0013] - paragraph [0046] * * figures 1,3,4,6 * ----- | 10-20 | |
| P,Y | US 2008/214934 A1 (LEE CHI-YIN [US] ET AL) 4 September 2008 (2008-09-04) * the whole document * ----- | 10-20 | |
| A | US 6 464 643 B1 (BROCK-FISHER GEORGE A [US]) 15 October 2002 (2002-10-15) * abstract * * column 2, line 46 - column 8, line 35; figures 1-4 * ----- | 10-20 | |
| A | US 2006/036174 A1 (GURACAR ISMAYIL M [US] ET AL) 16 February 2006 (2006-02-16) * the whole document * ----- | 11,12,18 | TECHNICAL FIELDS SEARCHED (IPC) A61B G06T G01S |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 6 February 2009 | Artikis, T |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04E07)

Europäisches
Patentamt

European
Patent Office

Office européen
des brevets

**INCOMPLETE SEARCH
SHEET C**

Application Number

EP 08 16 9745

Claim(s) not searched:
      1-9

Reason for the limitation of the search (non-patentable invention(s)):

Article 53 (c) EPC - Method for treatment of the human or animal body by surgery

**ANNEX TO THE EUROPEAN SEARCH REPORT
ON EUROPEAN PATENT APPLICATION NO.**
EP 08 16 9745

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2009

| Patent document cited in search report | | Publication date | Patent family member(s) | | Publication date |
|---|---|---|---|---|---|
| US 6659953 | B1 | 09-12-2003 | NONE | | |
| US 2005033179 | A1 | 10-02-2005 | US | 2008027319 A1 | 31-01-2008 |
| | | | US | 2008033294 A1 | 07-02-2008 |
| US 2008214934 | A1 | 04-09-2008 | WO | 2008108922 A1 | 12-09-2008 |
| US 6464643 | B1 | 15-10-2002 | NONE | | |
| US 2006036174 | A1 | 16-02-2006 | DE | 102005034697 A1 | 23-03-2006 |
| | | | US | 2006036175 A1 | 16-02-2006 |
| | | | US | 2009012400 A1 | 08-01-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6494841 B
- US 6602195 B
- US 6632177 B
- US 6638228 B
- US 6682482 B